# EUROPEAN PATENT SPECIFICATION

(11) **EP 3 757 949 B1**
(45) Date of publication and mention of the grant of the patent: **24.05.2023**
(21) Application number: 19912775.4
(22) Date of filing: 01.02.2019
(51) Int. Cl.: G07C 9/00, G07C 9/26, G07C 9/37, G06F 21/32, G06K 9/00

(54) **FINGERPRINT MODULE, FINGERPRINT RECOGNITION SYSTEM, CONTROL METHOD, AND SMART LOCK**
FINGERABDRUCKMODUL, FINGERABDRUCKERKENNUNGSSYSTEM, STEUERVERFAHREN UND INTELLIGENTES SCHLOSS
MODULE D'EMPREINTES DIGITALES, SYSTÈME DE RECONNAISSANCE D'EMPREINTES DIGITALES, PROCÉDÉ DE COMMANDE, ET SERRURE INTELLIGENTE

(43) Date of publication of application: 30.12.2020
(73) Proprietor: SHENZHEN GOODIX TECHNOLOGY CO., LTD., Shenzhen, Guangdong 518045 (CN)
(72) Inventor: LI, Qingbin, Shenzhen, Guangdong 518045 (CN); ZHOU, Luming, Shenzhen, Guangdong 518045 (CN)
(74) Representative: J A Kemp LLP
(86) International application number: PCT/CN2019/074496
(87) International publication number: WO 2020/155122

(56) References cited:
- WO-A1-2019/000294
- CN-A- 107 204 059
- CN-A- 107 544 798
- CN-A- 107 544 798
- CN-A- 107 820 598
- CN-A- 108 898 704
- CN-A- 108 961 492
- US-A1- 2013 263 252
- US-A1- 2016 086 010

## Description

### TECHNICAL FIELD

The present application relates to the field of fingerprint identification technologies, and in particular, to a fingerprint module, a fingerprint identification system, a control method and a smart lock.

### BACKGROUND

Smart locks are ubiquitous in people's lives, which can be unlocked in various ways, such as an unlocking method based on Near Field Communication (NFC), a digital password unlocking method, a fingerprint password unlocking method, etc. A smart lock can involve one or more unlocking methods.

At present, a smart lock with fingerprint identification function includes: a main control unit, a fingerprint sensor and a flash (FLASH). A fingerprint identification process of the smart lock is: the fingerprint sensor collects a fingerprint image and sends the fingerprint image to the main control unit, the main control unit matches the acquired fingerprint image with a fingerprint template in the flash to obtain a matching result, and when matching is successful, the main control unit in the smart lock controls an actuator to drive the smart lock to be in an open state.

In the prior art, the fingerprint identification process is mainly implemented by the main control unit, which will cause a problem of excessive power consumption of the main control unit.

US2016/0186010A1 discloses a system includes a fingerprint sensor and an auxiliary processor.

CN107544798A discloses a method for awakening an electronic device.

CN107204059A discloses a fingerprint identification system to solve the problem that the fingerprint recognition system in the prior art needs to be awakened by pressing the key when unlocking the smart lock.

### SUMMARY

The present invention is set out in the appended set of claims. The present application provides that power consumption of the smart lock can be reduced.

### BRIEF DESCRIPTION OF DRAWINGS

In order to describe the technical solutions in embodiments of the present application or in the prior art more clearly, the following briefly introduces the accompanying drawings needed for describing the embodiments or the prior art. Apparently, the accompanying drawings in the following description are merely some embodiments of the present application, and persons of ordinary skill in the art may still derive other accompanying drawings from these accompanying drawings without creative effort.
FIG. 1 is a schematic diagram of a fingerprint module according to an embodiment of the present application;
FIG. 2 is a schematic diagram of a fingerprint module according to another embodiment of the present application;
FIG. 3 is a schematic diagram of a fingerprint module according to a still another embodiment of the present application;
FIG. 4 is a schematic diagram of a fingerprint identification system according to an embodiment of the present application;
FIG. 5 is a schematic diagram of a fingerprint identification system according to another embodiment of the present application;
FIG. 6 is a schematic diagram of a fingerprint identification system according to still another embodiment of the present application;
FIG. 7 is a flowchart of a control method based on a fingerprint identification system according to an embodiment of the present application; and
FIG. 8 is a flowchart of a control method based on a fingerprint identification system according to another embodiment of the present application.

### DESCRIPTION OF EMBODIMENTS

In order to make the purposes, technical solutions and advantages in embodiments of the present application more clearly, the technical solutions in the embodiments of the present application will be described clearly and completely below in combination with accompanying drawings in the embodiments of the present application.

The terms "first", "second", "third", "fourth" and/or the like (if any) in the description, claims and the drawings of the present application are used to distinguish similar objects, and are not necessarily used to describe specific order or sequence. It should be understood that the numerals used as such are interchangeable under appropriate circumstances, so that the embodiments described herein, for example, can be implemented in alternative order other than those illustrated or described herein. In addition, the terms "include" and "have" and any variations thereof are intended to cover non-exclusive inclusion. For example, a process, a method, a system, a product or an apparatus that includes a series of steps or units do not have to be limited to those steps or units expressly listed, but may instead include other steps or units not expressly listed, or steps or units inherent to the process, method, product or apparatus.

As mentioned above, in the prior art, the fingerprint identification process is implemented by a main control unit, which will cause a problem of excessive power consumption of the main control unit. In order to solve this technical problem, the present application provides a fingerprint module, a fingerprint identification system, a control method and a smart lock.

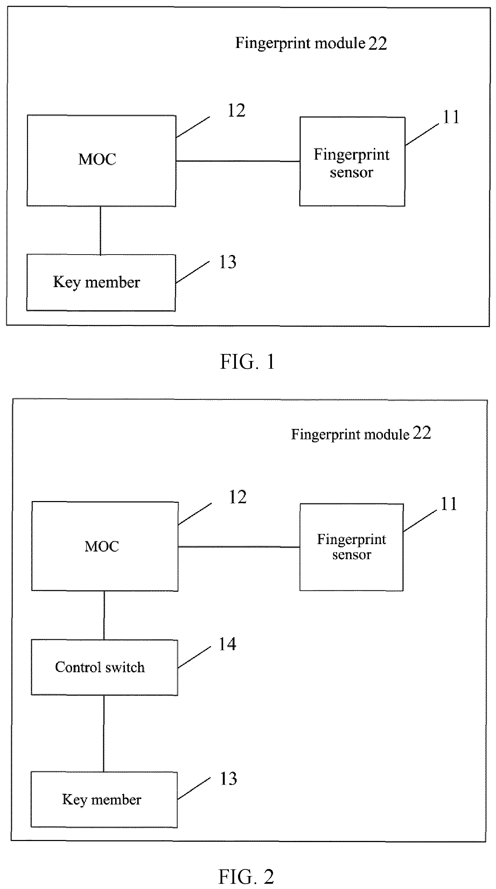

FIG. 1 is a schematic diagram of a fingerprint module according to an embodiment of the present application, and the fingerprint module is connected to a main control unit. As shown in FIG. 1, a fingerprint module 22 includes a fingerprint sensor 11, a match on chip (Match On Chip, MOC) 12 and a key member 13, and the key member 13 is connected to the fingerprint sensor 11. The fingerprint sensor 11 is configured to collect fingerprint information of a verification target and output the fingerprint information to the MOC 12. The MOC 12 is configured to authenticate the fingerprint information and output an authentication result to the main control unit.

The key member 13 is configured to generate an interrupt signal according to a touch operation of a user on the key member 13, to wake up the main control unit. When the main control unit does not receive information, for example, the authentication result sent by the MOC 12, within a preset period of time, the main control unit enters a sleep state. When the above fingerprint module is applied to a smart lock, power consumption of the smart lock can be reduced in this way. When the key member 13 acquires a touch operation of a user, the key member 13 generates an interrupt signal, which is used to wake up the main control unit, so that the main control unit enters a working state.

In a possible design, the main control unit may have a GPIO interface, and the key member 13 may transmit the interrupt signal to the main control unit through the GPIO interface.

When the main control unit is woken up, in this case, if the MOC 12 and/or fingerprint sensor 11 is in a power-off state, the main control unit sends a first control instruction to the MOC and/or fingerprint sensor to cause the MOC 12 and /or the fingerprint sensor 11 to enter a power-up state according to the first control instruction.

The MOC 12 is configured to control the key member 13 to be in the power-down state, when the MOC 12 is in the power-up state. Specifically, when the MOC 12 is in the power-up state, the MOC 12 performs a fingerprint identification process. In this case, the key member 13 does not need to work. In order to save the power consumption of the smart lock, the MOC 12 controls the key member 13 to be in a power-down state.

In a possible design, the main control unit may be a micro controller unit (Micro Controller Unit, MCU).

In a possible design, the main control unit has a universal asynchronous receiver transmitter (Universal Asynchronous Receiver Transmitter, UART) interface, and communicates with the MOC 12 through the UART interface.

In a possible design, there is a low dropout regulator (Low Dropout Regulator, LDO) switch connected between the main control unit and the fingerprint module, and the main control unit has a general purpose input output (General Purpose Input Output, GPIO) interface, and controls a state of the LDO switch through the GPIO interface.

In a possible design, the MOC 12 and the fingerprint sensor 11 are connected through a serial peripheral interface (Serial Peripheral Interface, SPI) interface, and the MOC 12 and the fingerprint sensor 11 can communicate through the SPI interface.

In a possible design, the key member 13 may be a ring key, which surrounds the fingerprint sensor 11. In a possible design, FIG. 2 is a schematic diagram of a fingerprint module according to another embodiment of the present application, where the MOC 12 is connected to the key member 13 through a control switch 14.

In a possible design, the MOC 12 is connected to the key member 13 through the control switch 14, and the MOC 12 is specifically configured to control the key member 13 to be in a power-down state through the control switch 14, when the MOC 12 is in a power-up state.

In a possible design, the MOC 12 has a GPIO interface, and the MOC 12 can control a state of the control switch 14 between the MOC 12 and the key member 13 through the GPIO interface.

The embodiment of the present application provides a fingerprint module, including: a fingerprint sensor, an MOC and a key member. The key member is connected to the fingerprint sensor. The fingerprint sensor is configured to collect fingerprint information of a verification target, and output the fingerprint information to the MOC. The MOC is configured to authenticate the fingerprint information, and output an authentication result to the main control unit. The key member is configured to generate an interrupt signal according to a touch operation of a user on the key member, to wake up the main control unit. That is, the MOC can implement a fingerprint identification process, so that power consumption of the main control unit can be reduced. When the MOC and/or the fingerprint sensor is in a power-off state, the main control unit sends a first control instruction to the MOC and/or the fingerprint sensor, so that the MOC and/or the fingerprint sensor enters a power-up state according to the first control instruction. Based on this, a smart lock including the fingerprint module can perform the fingerprint identification process, etc. The MOC is configured to control the key member to be in a power-down state, when the MOC 12 is in the power-up state. Therefore, when the fingerprint module is applied to the smart lock, power consumption of the smart lock can be reduced.

On the basis of the previous embodiment, further, when the main control unit does not receive information (the authentication result sent by the MOC 12) within a preset period of time, the main control unit sends a second control instruction to the MOC 12 and/or the fingerprint sensor 11, so that the MOC 12 and/or the fingerprint sensor 11 enters the power-down state according to the second control instruction. The above preset period of time may be set according to an actual condition, for example, the preset period of time is set to 10 minutes, 15 minutes, etc., which is not limited in the present application.

In a possible design, when the MOC 12 enters the power-down state, the MOC 12 is further configured to control the key member 13 to be in the power-up state.

In a possible design, the MOC 12 is connected to the key member 13 through the control switch 14; when the MOC 12 is in the power-down state, the MOC 12 is configured to trigger the control switch 14 to output a low-level signal, where the low-level signal is used to trigger the key member to be in the power-up state.

Or, in another possible design, when the MOC 12 is in the power-down state, the MOC 12 is configured to trigger the control switch 14 to output a high-level signal, where the high-level signal is used to trigger the key member to be in the power-up state.

In the above solution, the MOC 12 is configured to control the key member 13 to be in the power-up state. Actually, the main control unit may also directly control the key member 13 to be in the power-up state.

In a possible design, the main control unit is connected to the key member through the control switch. When the main control unit is in the sleep state, the main control unit triggers the control switch to output a low-level signal, where the low-level signal is used to trigger the key member to be in the power-up state.

Or, in another possible design, when the main control unit is in the sleep state, the main control unit triggers the control switch to output a high-level signal; where the high-level signal is used to trigger the key member to be in the power-up state.

The embodiment of the present application provides a fingerprint module, including: a fingerprint sensor, an MOC and a key member, and the key member is connected to the fingerprint sensor. When the main control unit does not receive information within a preset period of time, the main control unit sends a second control instruction to the MOC and/or fingerprint sensor, so that the MOC and/or the fingerprint sensor enters the power-down state according to the second control instruction. When the fingerprint module is applied to the smart lock, power consumption of the smart lock can be reduced. Optionally, when the MOC enters the power-down state, the MOC is further configured to control the key member to be in the power-up state, thereby ensuring that the smart lock can obtain an operation of a user at any time.

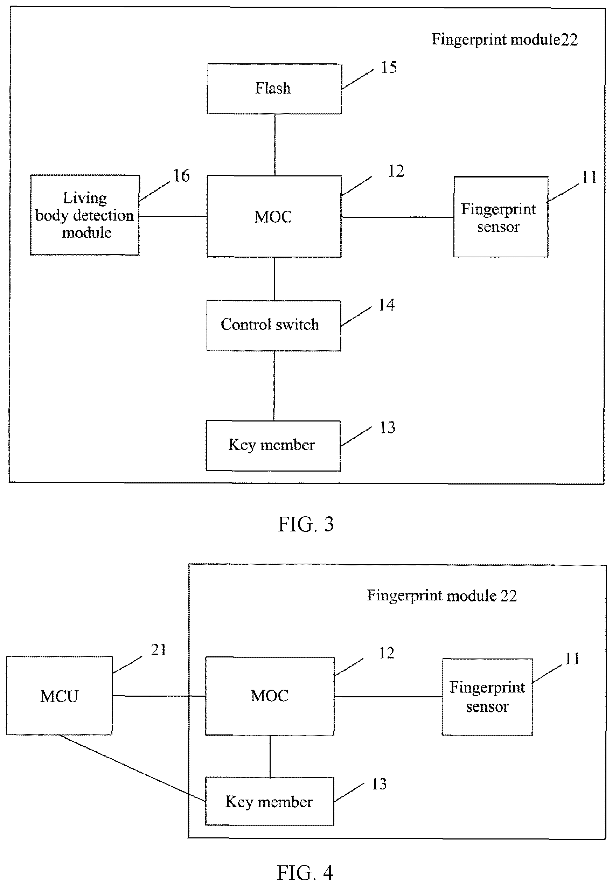

On the basis of any of the previous embodiments, further, the fingerprint module further includes: a flash (FLASH) and/or a living body detection module. The following is a detailed description of the fingerprint module including a flash and a living body detection module. FIG. 3 is a schematic diagram of a fingerprint module according to still another embodiment of the present application. As shown in FIG. 3, the fingerprint module includes: a flash 15 and a living body detection module 16, where the flash 15 includes at least one fingerprint template; and correspondingly, the MOC 12 is specifically configured to match the fingerprint information with the at least one fingerprint template, and output an authentication result to the main control unit. The authentication result is a result of successful authentication or a result of failed authentication.

In a possible design, the living body detection module is configured to detect at least one living body index of the verification target, and output the at least one living body index to the MOC. The MOC is further configured to determine whether the verification target is a living body according to the at least one living body index. The living body index includes at least one of: blood oxygen or heart rate. For example: when determining that a heart rate of the verification target is within a preset range, the MOC determines the verification target is a living body.

When the fingerprint information is successfully authenticated and the verification target is the living body, the MOC may output an authentication result of successful authentication to the main control unit. Conversely, when the fingerprint information fails to be authenticated or the verification target is not the living body, the MOC may output an authentication result of failed authentication to the main control unit.

After the main control unit is woken up, if at least one of the MOC 12, the fingerprint sensor 11, the flash 15 and the living body detection module 16 is in the power-off state, the main control unit will send a first control instruction to at least one of the MOC 12, the fingerprint sensor 11, the flash 15 and the living body detection module 16, so that the at least one of the MOC 12, the fingerprint sensor 11, the flash 15 and the living body detection module 16 will enter the power-up state according to the first control instruction.

When the main control unit does not receive information (the authentication result sent by the MOC 12) within a preset period of time, and at least one of the MOC 12, the fingerprint sensor 11, the flash 15 and the living body detection module 16 is in the power-up state, the main control unit sends a second control instruction at least one of the MOC 12, the fingerprint sensor 11, the flash 15 and the living body detection module 16, so that at least one of the MOC 12, the fingerprint sensor 11, the flash 15 and the living body detection module 16 enters the power-down state according to the second control instruction.

The embodiment of the present application provides a fingerprint module, which includes a fingerprint sensor, an MOC, a key member, a FLASH and a living body detection module. After the main control unit is woken up, the main control unit may send a first control instruction to at least one of the MOC, the fingerprint sensor, the flash and the living body detection module, so that at least one of the MOC, the fingerprint sensor, the flash and the living body detection module will enter the power-up state according to the first control instruction. Based on this, a smart lock including the fingerprint module can perform a fingerprint identification process, etc. The MOC is configured to control the key member to be in a power-down state, when the MOC is in the power-up state, which can further reduce power consumption of the smart lock. When the main control unit does not receive information within a preset period of time, the main control unit will send a second control instruction to at least one of the MOC, the fingerprint sensor, the flash and the living body detection module, so that at least one of the MOC, the fingerprint sensor, the flash and the living body detection module enters the power-down state according to the second control instruction. When the fingerprint module is applied to the smart lock, power consumption of the smart lock can be reduced. When the MOC enters the power-down state, the MOC is further configured to control the key member to be in the power-up state, thereby ensuring that the smart lock can obtain an operation of a user at any time.

FIG. 4 is a schematic diagram of a fingerprint identification system according to an embodiment of the present application, where the fingerprint identification system may be applied to but not limited to a smart lock. As shown in FIG. 4, the fingerprint identification system includes: a main control unit 21 and a fingerprint module 22. The fingerprint module 22 includes a fingerprint sensor 11, a MOC 12 and a key member 13, and the key member 13 is connected to the fingerprint sensor 11. The fingerprint sensor 11 is configured to collect fingerprint information of a verification target and output the fingerprint information to the MOC 12. The MOC 12 is configured to authenticate the fingerprint information and output an authentication result to the main control unit 21.

The key member 13 is configured to generate an interrupt signal according to a touch operation of a user on the key member 13, to wake up the main control unit 21. When the main control unit 21 does not receive information (the authentication result sent by the MOC 12) within a preset period of time, the main control unit 21 enters a sleep state. When the above fingerprint module is applied to a smart lock, power consumption of the smart lock can be reduced in this way. However, when the key member 13 acquires the touch operation of a user, the key member 13 generates an interrupt signal, and the interrupt signal is used to wake up the main control unit 21 so that the main control unit 21 enters a working state.

In a possible design, the main control unit 21 may have a GPIO interface, and the key member 13 may transmit the interrupt signal to the main control unit 21 through the GPIO interface.

When the main control unit 21 is woken up, in this case, if the MOC 12 and/or fingerprint sensor 11 is in a power-off state, the main control unit 21 sends a first control instruction to the MOC and/or fingerprint sensor, so that the MOC 12 and /or the fingerprint sensor 11 enters a power-up state according to the first control instruction.

The MOC 12 is configured to control the key member 13 to be in the power-down state, when the MOC 12 is in the power-up state. Specifically, when the MOC 12 is in the power-up state, the MOC 12 performs a fingerprint identification process. In this case, the key member 13 does not need to work. In order to save the power consumption of the smart lock, the MOC 12 controls the key member 13 to be in a power-down state.

In a possible design, the main control unit 21 may be a MCU.

In a possible design, the main control unit 21 has a UART interface, and communicates with the MOC 12 through the UART interface.

In a possible design, there is a LDO switch connected between the main control unit 21 and the fingerprint module, and the main control unit 21 has a GPIO interface, and controls a state of the LDO switch through the GPIO interface.

In a possible design, the MOC 12 and the fingerprint sensor 11 are connected through an SPI interface, and the MOC 12 and the fingerprint sensor 11 can communicate through the SPI interface.

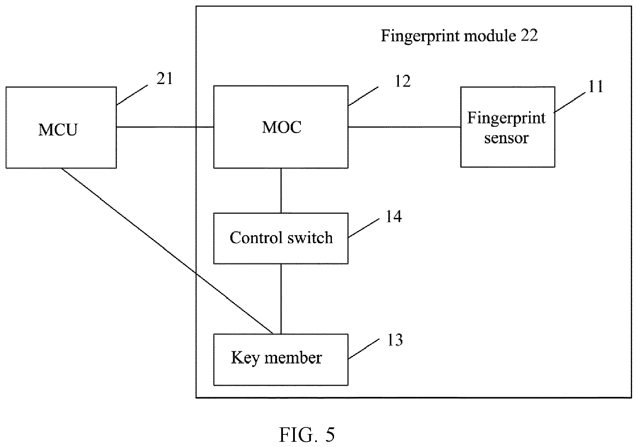

In a possible design, the key member 13 may be a ring key, which surrounds the fingerprint sensor 11. In a possible design, FIG. 5 is a schematic diagram of a fingerprint identification system according to another embodiment of the present application, where the MOC 12 is connected to the key member 13 through a control switch 14 as shown in FIG. 5.

In a possible design, the MOC 12 is connected to the key member 13 through the control switch 14. The MOC 12 is specifically configured to control the key member 13 to be in a power-down state through the control switch 14, when the MOC is in a power-up state.

In a possible design, the MOC 12 has a GPIO interface, and the MOC 12 can control a state of the control switch 14 between the MOC 12 and the key member 13 through the GPIO interface.

The embodiment of the present application provides a fingerprint identification system, where the fingerprint identification system includes the fingerprint module described in the above embodiments, and reference can be made to the embodiments of the fingerprint module for effects of the fingerprint identification system, which will not be repeated here.

On the basis of the previous embodiment, further, when the main control unit does not receive information (the authentication result sent by the MOC 12) within a preset period of time, the main control unit sends a second control instruction to the MOC 12 and/or the fingerprint sensor 11, so that the MOC 12 and/or the fingerprint sensor 11 enters the power-down state according to the second control instruction. The above preset period of time can be set according to an actual condition, for example, the preset period is set to 10 minutes, 15 minutes, etc., which is not limited in the present application.

In a possible design, when the MOC 12 enters the power-down state, the MOC 12 is further configured to control the key member 13 to be in the power-up state.

In a possible design, the MOC 12 is connected to the key member 13 through the control switch 14, and when the MOC 12 is in the power-down state, the MOC 12 is configured to trigger the control switch 14 to output a low-level signal, where the low-level signal is used to trigger the key member to be in the power-up state.

Or, in another possible design, when the MOC 12 is in the power-down state, the MOC 12 is configured to trigger the control switch 14 to output a high-level signal; where the high-level signal is used to trigger the key member to be in the power-up state.

In the above solution, the MOC 12 is configured to control the key member 13 to be in the power-up state. Actually, the main control unit may also directly control the key member 13 to be in the power-up state.

In a possible design, the main control unit is connected to the key member through the control switch, and when the main control unit is in the sleep state, the main control unit triggers the control switch to output a low-level signal, where the low-level signal is used to trigger the key member to be in the power-up state.

Or, in another possible design, when the main control unit is in the sleep state, the main control unit triggers the control switch to output a high-level signal, where the high-level signal is used to trigger the key member to be in the power-up state.

The embodiment of the present application provides a fingerprint identification system, where the fingerprint identification system includes the fingerprint module described in the above embodiments, and reference can be made to the embodiments of the fingerprint module for effects of the fingerprint identification system, which will not be repeated here.

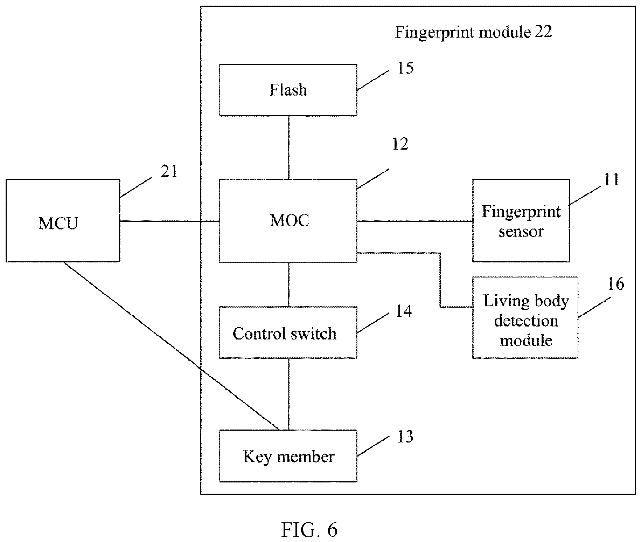

On the basis of any of the previous embodiments, further, the fingerprint module further includes: a flash and/or a living body detection module. The following is a detailed description of the fingerprint module including a flash and/or a living body detection module. FIG. 6 is a schematic diagram of a fingerprint identification system according to still another embodiment of the present application. As shown in FIG. 6, the fingerprint module in the system further includes: a flash 15 and a living body detection module 16. The flash 15 includes at least one fingerprint template, and correspondingly, the MOC 12 is specifically configured to match the fingerprint information with the at least one fingerprint template, and output an authentication result to the main control unit. The authentication result is a result of successful authentication or a result of failed authentication.

In a possible design, the living body detection module 16 is configured to detect at least one living body index of the verification target, and output the at least one living body index to the MOC 12. The MOC 12 is further configured to determine whether the verification target is a living body according to the at least one living body index. The living body index includes at least one of: blood oxygen or heart rate. For example: when determining that a heart rate of the verification target is within a preset range, the MOC 12 determines the verification target is a living body.

When the fingerprint information is successfully authenticated and the verification target is the living body, the MOC 12 may output an authentication result of successful authentication to the main control unit 21. Conversely, when the fingerprint information fails to be authenticated or the verification target is not the living body, the MOC 12 may output an authentication result of failed authentication to the main control unit.

After the main control unit 21 is woken up, in this case, if at least one of the MOC 12, the fingerprint sensor 11, the flash 15 and the living body detection module 16 is in the power-off state, the main control unit 21 will send a first control instruction to at least one of the MOC 12, the fingerprint sensor 11, the flash 15 and the living body detection module 16, so that at least one of the MOC 12, the fingerprint sensor 11, the flash 15 and the living body detection module 16 will enter the power-up state according to the first control instruction.

When the main control unit 21 does not receive information, (the authentication result sent by the MOC 12) within a preset period of time, and at least one of the MOC 12, the fingerprint sensor 11, the flash 15 and the living body detection module 16 is in the power-up state, the main control unit 21 sends a second control instruction to at least one of the MOC 12, the fingerprint sensor 11, the flash 15 and the living body detection module 16, so that at least one of the MOC 12, the fingerprint sensor 11, the flash 15 and the living body detection module 16 enters the power-down state according to the second control instruction.

The embodiment of the present application provides a fingerprint identification system, where the fingerprint identification system includes the fingerprint module described in the above embodiments, and reference can be made to the embodiments of the fingerprint module for effects of the fingerprint identification system, which will not be repeated here.

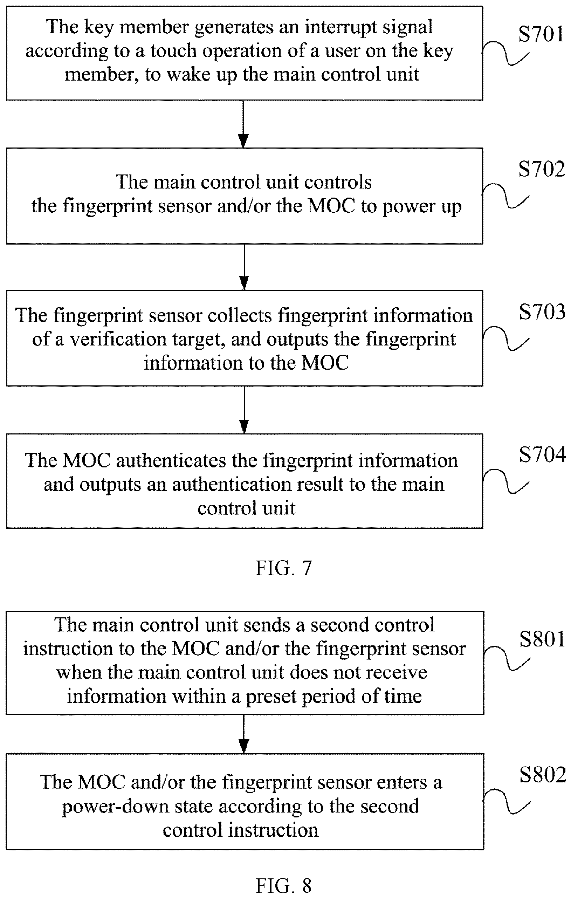

FIG. 7 is a flowchart of a control method based on a fingerprint identification system according to a non-claimed embodiment of the present application. The fingerprint identification system includes: a main control unit and a fingerprint module. The fingerprint module includes: a fingerprint sensor, a match on chip (MOC) and a key member. The key member is connected to the fingerprint sensor. As shown in FIG.7, the method includes the following process:
Step S701: The key member generates an interrupt signal according to a touch operation of a user on the key member, to wake up the main control unit.
Step S702: The main control unit controls the fingerprint sensor and/or the MOC to power up.
Step S703: The fingerprint sensor collects fingerprint information of a verification target, and outputs the fingerprint information to the MOC.
Step S704: The MOC authenticates the fingerprint information and outputs an authentication result to the main control unit.
Step S702 includes: the main control unit sends a first control instruction to the MOC and/or the fingerprint sensor, so that the MOC and/or the fingerprint sensor enters a power-up state according to the first control instruction.

The method further includes: the MOC controls the key member to be in a power-down state, when the MOC is in a power-up state.

In a possible design, the MOC is connected to the key member through a control switch; and the MOC controlling, the key member to be in a power-down state includes: the MOC controls the key member to be in the power-down state through the control switch, when the MOC is in the power-up state.

The method may be executed by the above fingerprint identification system, and for contents and effects thereof, reference may be made to the embodiments of the above fingerprint identification system, which will not be repeated here.

FIG. 8 is a flowchart of a control method based on a fingerprint identification system according to another non-claimed

embodiment of the present application. The fingerprint identification system includes: a main control unit and a fingerprint module. The fingerprint module includes: a fingerprint sensor, an MOC and a key member. The key member is connected to the fingerprint sensor. As shown in FIG.8, the method includes the following process:
Step S801: The main control unit sends a second control instruction to the MOC and/or the fingerprint sensor when the main control unit does not receive information within a preset period of time.
Step S802: The MOC and/or the fingerprint sensor enters a power-down state according to the second control instruction.

In a possible design, when the MOC is in the power-down state, the method further includes: the MOC controls the key member to be in a power-up state.

In a possible design, the MOC is connected to the key member through a control switch, and the MOC controlling the key member to be in a power-up state includes: the MOC triggers the control switch to output a low-level signal when the MOC is in the power-down state, where the low-level signal is used to trigger the key member to be in the power-up state.

In a possible design, the fingerprint module further includes: a flash; the flash includes at least one fingerprint template; the MOC authenticating the fingerprint information and outputting the authentication result to the main control unit includes: the MOC matches the fingerprint information with the at least one fingerprint template, and outputs the authentication result.

In a possible design, the authentication result is a result of successful authentication or a result of failed authentication.

In a possible design, the fingerprint module further includes a living body detection module; and correspondingly, the method further includes: the living body detection module detects at least one living body index of the verification target, and outputs the at least one living body index to the MOC; and the MOC determines whether the verification target is a living body according to the at least one living body index.

In a possible design, there further includes: the MOC outputs an authentication result of successful authentication when the fingerprint information is successfully authenticated and the verification target is a living body.

In a possible design, the living body index includes at least one of: blood oxygen or heart rate.

The method may be performed by the above fingerprint identification system, and for contents and effects thereof, reference may be made to the embodiments of the above fingerprint identification system, which will not be repeated here.

The present application further provides a smart lock, including: the above fingerprint identification system. For functions and effects of the system, reference may be made to the above embodiments, which will not be repeated in the present application.

It should be noted that an unlocking method of the smart lock may include a fingerprint password recognition method. Optionally, the smart lock may further include at least one unlocking method as follows: an unlocking method based on NFC, a digital password unlocking method, and the like.

Persons of ordinary skill in the art could understand that all or part of the steps in the above method embodiments may be implemented by a program instructing relevant hardware. The foregoing program may be stored in a computer readable storage medium. When the program is executed, the steps including the above method embodiments are executed. The foregoing storage medium may include a ROM, a RAM, a magnetic disk, or an optical disk and other media that can store program codes.

## Claims

1. A fingerprint identification system, comprising: a main control unit (21) and a fingerprint module (22), wherein the fingerprint module (22) is connected to the main control unit (21), and the fingerprint module (22) comprises: a fingerprint sensor (11), a match on chip, MOC, (12) and a key member (13), and the key member (13) is connected to the fingerprint sensor (11);
the key member (13) is configured to generate an interrupt signal according to a touch operation of a user on the key member (13), to wake up the main control unit (21);
the MOC (12) and/or the fingerprint sensor (11), when in a power-off state, is configured to receive a first control instruction sent by the main control unit (21), and enter a power-up state according to the first control instruction;
the fingerprint sensor (11) is configured to collect fingerprint information of a verification target, and output the fingerprint information to the MOC (12);
the MOC (12) is configured to authenticate the fingerprint information, and output an authentication result to the main control unit (21);
**characterized in that** when the MOC (12) performs a fingerprint identification process, the MOC (12) is further configured to control the key member (13) to be in a power-down state; and
when the main control unit (21) does not receive the authentication result sent by the MOC (12) within a preset period of time, and at least one of the MOC (12) and/or the fingerprint sensor (11) is in the power-up state, the MOC (12) and/or the fingerprint sensor (11) is further configured to receive a second control instruction sent by the main control unit (21), and enter the power-down state according to the second control instruction.

2. The fingerprint identification system according to claim 1, wherein the MOC (12) is connected to the key member (13) through a control switch (14); and
the MOC (12) is specifically configured to control the key member (13) to be in the power-down state through the control switch (14) when the MOC (12) is in the power-up state.

3. The fingerprint identification system according to claim 1, wherein:
the MOC (12) is further configured to control the key member (13) to be in a power-up state.

4. The fingerprint identification system according to claim 3, wherein the MOC (12) is connected to the key member (13) through a control switch (14); and
the MOC (12) is configured to trigger the control switch (14) to output a low-level signal, when the MOC (12) is in the power-down state;
wherein the low-level signal is used to trigger the key member (13) to be in the power-up state.

5. The fingerprint identification system according to any one of claims 1-4, further comprising: a flash (15);
the flash (15) comprising at least one fingerprint template;
the MOC (12) being specifically configured to match the fingerprint information with the at least one fingerprint template, and output the authentication result.

6. The fingerprint identification system according to claim 5, wherein:
the authentication result is a result of successful authentication or a result of failed authentication.

7. The fingerprint identification system according to claim 1, wherein the fingerprint module (22) further comprises a living body detection module (16);
the living body detection module (16) is configured to detect at least one living body index of the verification target, and output the at least one living body index to the MOC (12); and
the MOC (12) is further configured to determine whether the verification target is a living body according to the at least one living body index.

8. The fingerprint identification system according to claim 7, wherein the MOC (12) is configured to output an authentication result of successful authentication when the fingerprint information is successfully authenticated and the verification target is the living body, and the living body index comprises at least one of: blood oxygen or heart rate.

9. A control method for a fingerprint identification system, wherein the fingerprint identification system comprises: a main control unit and a fingerprint module, and the fingerprint module comprises: a fingerprint sensor, a match on chip, MOC, and a key member, and the key member is connected to the fingerprint sensor; and correspondingly, the control method comprises:
generating (S701), by the key member, an interrupt signal according to a touch operation of a user on the key member, to wake up the main control unit;
sending, by the main control unit, a first control instruction to the MOC and/or the fingerprint sensor when the MOC and/or the fingerprint sensor is in a power-off state, so that the MOC and/or the fingerprint sensor enters a power-up state according to the first control instruction;
collecting (S703), by the fingerprint sensor, fingerprint information of a verification target, and outputting the fingerprint information to the MOC;
authenticating (S704), by the MOC, the fingerprint information and outputting an authentication result to the main control unit;
**characterized in that** controlling, by the MOC, the key member to be in a power-down state, when the MOC performs a fingerprint identification process; and
when the main control unit does not receive the authentication result sent by the MOC within a preset period of time, and at least one of the MOC and/or the fingerprint sensor is in the power-up state, sending, by the main control unit, a second control instruction to the MOC and/or the fingerprint sensor, and entering, by the MOC and/or the fingerprint sensor, the power-down state according to the second control instruction.

10. A smart lock, **characterized by** comprising: the fingerprint identification system according to any one of claims 1-8.

## Patentansprüche

1. Fingerabdruckidentifikationssystem, umfassend: eine Hauptsteuereinheit (21) und ein Fingerabdruckmodul (22), wobei das Fingerabdruckmodul (22) mit der Hauptsteuereinheit (21) verbunden ist und das Fingerabdruckmodul (22) umfasst: einen Fingerabdrucksensor (11), einen Match-on-Chip, MOC, (12) und ein Tastenelement (13), und das Tastenelement (13) mit dem Fingerabdrucksensor (11) verbunden ist;
das Tastenelement (13) dazu konfiguriert ist, ein Unterbrechungssignal gemäß einer Berührungsoperation eines Benutzers auf dem Tastenelement (13) zu erzeugen, um die Hauptsteuereinheit (21) aufzuwecken;
der MOC (12) und/oder der Fingerabdrucksensor (11), wenn er sich in einem ausgeschalteten Zustand befindet, dazu konfiguriert ist, eine erste Steueranweisung zu empfangen, die von der Hauptsteuereinheit (21) gesendet wird, und gemäß der ersten Anweisung in einen Einschaltzustand einzutreten;
der Fingerabdrucksensor (11) dazu konfiguriert ist, Fingerabdruckinformationen eines Verifizierungsziels zu sammeln und die Fingerabdruckinformationen an den MOC (12) auszugeben;
der MOC (12) dazu konfiguriert ist, die Fingerabdruckinformationen zu authentifizieren und ein Authentifizierungsergebnis an die Hauptsteuereinheit (21) auszugeben;
**dadurch gekennzeichnet, dass**, wenn der MOC (12) einen Fingerabdruckidentifikationsprozess durchführt, der MOC (12) ferner dazu konfiguriert ist, das Tastenelement (13) zu steuern, um sich in einem Abschaltzustand zu befinden; und
wenn die Hauptsteuereinheit (21) das von dem MOC (12) gesendete Authentifizierungsergebnis nicht innerhalb einer voreingestellten Zeitspanne empfängt und sich wenigstens einer des MOC (12) und/oder des Fingerabdrucksensors (11) in dem Einschaltzustand befindet, der MOC (12) und/oder der Fingerabdrucksensor (11) ferner dazu konfiguriert ist, eine zweite Steueranweisung zu empfangen, die von der Hauptsteuereinheit (21) gesendet wird, und gemäß der zweiten Steueranweisung in den Ausschaltzustand einzutreten.

2. Fingerabdruckidentifikationssystem nach Anspruch 1, wobei der MOC (12) mit dem Tastenelement (13) über einen Steuerschalter (14) verbunden ist; und
der MOC (12) speziell dazu konfiguriert ist, das Tastenelement (13) über den Steuerschalter (14) zu steuern, um sich in dem ausgeschalteten Zustand zu befinden, wenn sich der MOC (12) in dem eingeschalteten Zustand befindet.

3. Fingerabdruckidentifikationssystem nach Anspruch 1, wobei:
der MOC (12) ferner dazu konfiguriert ist, das Tastenelement (13) zu steuern, sich in einem Einschaltzustand zu befinden.

4. Fingerabdruckidentifikationssystem nach Anspruch 3, wobei der MOC (12) mit dem Tastenelement (13) durch einen Steuerschalter (14) verbunden ist; und
der MOC (12) dazu konfiguriert ist, den Steuerschalter (14) auszulösen, um ein Signal mit niedrigem Pegel auszugeben, wenn sich der MOC (12) in dem ausgeschalteten Zustand befindet;
wobei das Signal mit niedrigem Pegel verwendet wird, um das Tastenelement (13) auszulösen, sich in dem eingeschalteten Zustand zu befinden.

5. Fingerabdruckidentifikationssystem nach einem der Ansprüche 1-4, das ferner umfasst: einen Flash (15);
wobei der Flash (15) wenigstens eine Fingerabdruckvorlage umfasst;
wobei der MOC (12) speziell dazu konfiguriert ist, die Fingerabdruckinformation mit der wenigstens einen Fingerabdruckvorlage abzugleichen und das Authentifizierungsergebnis auszugeben.

6. Fingerabdruckidentifikationssystem nach Anspruch 5, wobei:
das Authentifizierungsergebnis ein Ergebnis einer erfolgreichen Authentifizierung oder ein Ergebnis einer fehlgeschlagenen Authentifizierung ist.

7. Fingerabdruckidentifikationssystem nach Anspruch 1, wobei das Fingerabdruckmodul (22) ferner ein Modul (16) für die Erkennung lebender Körper umfasst;
das Modul (16) für die Erkennung lebender Körper dazu konfiguriert ist, wenigstens einen Lebendkörperindex des Verifikationsziels zu erkennen und den wenigstens einen Lebendkörperindex an den MOC (12) auszugeben; und
der MOC (12) dazu konfiguriert ist, um zu bestimmen, ob das Verifizierungsziel gemäß dem wenigstens einen Lebendkörperindex ein Lebendkörper ist.

8. Fingerabdruckidentifikationssystem nach Anspruch 7, wobei der MOC (12) dazu konfiguriert ist, ein Authentifizierungsergebnis einer erfolgreichen Authentifizierung auszugeben, wenn die Fingerabdruckinformationen erfolgreich authentifiziert wurden und das Verifizierungsziel der lebende Körper ist, und der Lebendkörperindex wenigstens eines umfasst von: Blutsauerstoff oder Herzfrequenz.

9. Steuerverfahren für ein Fingerabdruckidentifikationssystem, wobei das Fingerabdruckidentifikationssystem umfasst: eine Hauptsteuereinheit und ein Fingerabdruckmodul, und das Fingerabdruckmodul umfasst: einen Fingerabdrucksensor, ein Match-on-Chip, MOC, und ein Tastenelement, und das Tastenelement mit dem Fingerabdrucksensor verbunden ist; und dementsprechend das Steuerverfahren umfasst:
Erzeugen (S701) durch das Tastenelement eines Unterbrechungssignals gemäß einer Berührungsoperation eines Benutzers auf dem Tastenelement, um die Hauptsteuereinheit aufzuwecken;
Senden durch die Hauptsteuereinheit einer ersten Steueranweisung an den MOC und/oder den Fingerabdrucksensor, wenn sich der MOC und/oder der Fingerabdrucksensor in einem Ausschaltzustand befindet, sodass der MOC und/oder der Fingerabdrucksensor gemäß der ersten Steueranweisung in einen Einschaltzustand eintritt;
Sammeln (S703) durch den Fingerabdrucksensor von Fingerabdruckinformationen eines Verifizierungsziels und Ausgeben der Fingerabdruckinformationen an den MOC;
Authentifizierung (S704) durch den MOC der Fingerabdruckinformationen und Ausgeben eines Authentifizierungsergebnisses an die Hauptsteuereinheit;
**dadurch gekennzeichnet, dass** das Steuern durch den MOC des Tastenelements, sich in einem Abschaltzustand zu befinden, wenn der MOC einen Fingerabdruckidentifikationsprozess durchführt; und
wenn die Hauptsteuereinheit das von dem MOC gesendete Authentifizierungsergebnis nicht innerhalb einer voreingestellten Zeitspanne empfängt und wenigstens einer des MOC und/oder des Fingerabdrucksensors sich in dem Einschaltzustand befindet, Senden durch die Hauptsteuereinheit einer zweiten Steueranweisung an den MOC und/oder den Fingerabdrucksensor und Eintreten durch den MOC und/oder den Fingerabdrucksensor in den Ausschaltzustand gemäß der zweiten Steueranweisung.

10. Intelligentes Schloss, **gekennzeichnet durch** das Umfassen von: dem Fingerabdruckidentifikationssystem nach einem der Ansprüche 1-8.

## Revendications

1. Système d'identification d'empreintes digitales, comprenant : une unité de commande principale (21) et un module d'empreintes digitales (22), dans lequel le module d'empreintes digitales (22) est connecté à l'unité de commande principale (21), et le module d'empreintes digitales (22) comprend : un capteur d'empreintes digitales (11), une correspondance sur puce, MOC, (12) et un élément de touche (13), et l'élément de touche (13) est connecté au capteur d'empreintes digitales (11) ;
l'élément de touche (13) est configuré pour générer un signal d'interruption selon une opération tactile d'un utilisateur sur l'élément de touche (13), pour réveiller l'unité de commande principale (21) ;
la MOC (12) et/ou le capteur d'empreintes digitales (11), lorsqu'ils sont dans un état hors tension, sont configurés pour recevoir une première instruction de commande envoyée par l'unité de commande principale (21), et entrent dans un état de mise sous tension selon la première instruction de commande ;
le capteur d'empreintes digitales (11) est configuré pour collecter des informations d'empreintes digitales d'une cible de vérification, et sortir les informations d'empreintes digitales à la MOC (12);
la MOC (12) est configurée pour authentifier les informations d'empreintes digitales et sortir un résultat d'authentification à l'unité de commande principale (21) ;
**caractérisé en ce que** lorsque la MOC (12) exécute un processus d'identification d'empreintes digitales, la MOC (12) est en outre configurée pour commander l'élément de touche (13) pour qu'il soit dans un état de veille ; et
lorsque l'unité de commande principale (21) ne reçoit pas le résultat d'authentification envoyé par le MOC (12) dans un délai prédéfini, et au moins l'un parmi la MOC (12) et/ou le capteur d'empreintes digitales (11) est dans le état de mise sous tension, la MOC (12) et/ou le capteur d'empreintes digitales (11) est en outre configuré pour recevoir une deuxième instruction de commande envoyée par l'unité de commande principale (21), et entrer dans l'état de vaille selon la deuxième instruction de commande.

2. Système d'identification d'empreintes digitales selon la revendication 1, dans lequel la MOC (12) est connectée à l'élément de touche (13) par l'intermédiaire d'un commutateur de commande (14) ; et
la MOC (12) est spécifiquement configuré pour commander l'élément de touche (13) pour qu'il soit dans l'état de veille par l'intermédiaire du commutateur de commande (14) lorsque la MOC (12) est dans l'état de mise sous tension.

3. Système d'identification d'empreintes digitales selon la revendication 1, dans lequel :
la MOC (12) est en outre configuré pour commander l'élément de touche (13) pour qu'il soit dans un état de mise sous tension.

4. Système d'identification d'empreintes digitales selon la revendication 3, dans lequel la MOC (12) est connectée à l'élément de touche (13) par l'intermédiaire d'un commutateur de commande (14) ; et
la MOC (12) est configurée pour déclencher le commutateur de commande (14) pour émettre un signal de bas niveau, lorsque la MOC (12) est dans l'état de veille ;
dans lequel le signal de bas niveau est utilisé pour déclencher l'élément de touche (13) pour qu'il soit dans l'état de mise sous tension.

5. Système d'identification d'empreintes digitales selon l'une quelconque des revendications 1 à 4, comprenant en outre : un flash (15) ;
le flash (15) comprenant au moins un modèle d'empreintes digitales ;
la MOC (12) étant spécifiquement configurée pour faire correspondre les informations d'empreintes digitales avec l'au moins un modèle d'empreintes digitales, et sortir le résultat d'authentification.

6. Système d'identification d'empreintes digitales selon la revendication 5, dans lequel :
le résultat de l'authentification est le résultat d'une authentification réussie ou d'un échec d'authentification.

7. Système d'identification d'empreintes digitales selon la revendication 1, dans lequel le module d'empreintes digitales (22) comprend en outre un module de détection de corps vivant (16) ;
le module de détection de corps vivant (16) est configuré pour détecter au moins un indice de corps vivant de la cible de vérification, et sortir l'au moins un indice de corps vivant à la MOC (12) ; et
la MOC (12) est en outre configurée pour déterminer si la cible de vérification est un corps vivant selon au moins un indice de corps vivant.

8. Système d'identification d'empreintes digitales selon la revendication 7, dans lequel la MOC (12) est configurée pour produire un résultat d'authentification réussie lorsque les informations d'empreintes digitales sont authentifiées avec succès et la cible de vérification est le corps vivant, et l'indice de corps vivant comprend au moins l'un parmi : l'oxygène sanguin ou la fréquence cardiaque.

9. Procédé de commande pour un système d'identification d'empreintes digitales, dans lequel le système d'identification d'empreintes digitales comprend : une unité de commande principale et un module d'empreintes digitales, et le module d'empreintes digitales comprend : un capteur d'empreintes digitales, une correspondance sur puce, MOC, et un élément de touche, et l'élément de touche est connecté au capteur d'empreintes digitales ; et de manière correspondante, le procédé de commande comprend :
la génération (S701), par l'élément de touche, d'un signal d'interruption selon une opération tactile d'un utilisateur sur l'élément de touche, pour réveiller l'unité de commande principale ;
l'envoi, par l'unité de commande principale, d'une première instruction de commande à la MOC et/ou au capteur d'empreintes digitales lorsque la MOC et/ou le capteur d'empreintes digitales est dans un état hors tension, de sorte que la MOC et/ou le capteur d'empreintes digitales entre dans un état de mise sous tension selon la première instruction de commande ;
la collecte (S703), par le capteur d'empreintes digitales, d'informations d'empreintes digitales d'une cible de vérification, et la sortie des informations d'empreintes digitales à la MOC ;
l'authentification (S704), par la MOC, des informations d'empreintes digitales et la sortie d'un résultat d'authentification à l'unité de commande principale ;
**caractérisé en ce que** la commande, par la MOC, de l'élément de touche pour qu'il soit dans un état de veille, lorsque la MOC exécute un processus d'identification d'empreintes digitales ; et
lorsque l'unité de commande principale ne reçoit pas le résultat d'authentification envoyé par la MOC dans un délai prédéfini, et au moins l'un parmi la MOC et/ou le capteur d'empreintes digitales est dans l'état de mise sous tension, l'envoi, par l'unité de commande principale, d'une deuxième instruction de commande à la MOC et/ou au capteur d'empreintes digitales, et l'entrée, par la MOC et/ou le capteur d'empreintes digitales, à l'état de veille selon la deuxième instruction de commande.

10. Serrure intelligente, **caractérisée en ce qu'**elle comprend : le système d'identification d'empreintes digitales selon l'une quelconque des revendications 1 à 8.
